# EUROPEAN PATENT APPLICATION

(11) **EP 0 982 045 A2**
(43) Date of publication of application: **01.03.2000**
(21) Application number: 99306871.7
(22) Date of filing: 27.08.1999
(51) Int. Cl.: A61M 16/04

(54) **An airway bite protector**

(30) Priority: 27.08.1998 GB 9818558
(71) Applicant: Craft, Timothy Michael, Bath BA2 4QR (GB); Hughes, John J, Mangotsfield, Bristol BS17 3NH (GB)
(72) Inventor: Craft, Timothy Michael, Dr., Bath BA2 4QR (GB)
(74) Representative: Bryer, Kenneth Robert

(57) **Abstract**

A bite protector for an airway comprises an elongate body (18) having a longitudinally extending passage (10) therethrough and a discontinuity (23) along the wall at one side of the body and a hinge (24) along the other. A flange (20) is provided at one end to limit the distance the bite protector (18) can be introduced into the mouth of a patient and a reinforced wall portion (19) at least in the region adjacent the flange (20) is provided for resisting the bite reaction of a patient upon recovery from anaesthesia. The hinge (24) is such as to allow the body (18) to be opened laterally to an extent such that the opposite edges of the discontinuity (23) are spaced from one another by a distance at least as great as the maximum diameter of the passage (10) through the body (18) when closed.

## Description

The present invention relates generally to oral airways for medical practices, and particularly to an improved oral airway having means for protecting reusable parts of the equipment from damage which may be inflicted by the patient.

The introduction of oral airways, often referred to as intubation may be required for various reasons although most frequently it is required to enable a medical practitioner to administer air or oxygen (or other gas) when a patient is under sedation or anaesthetic. When a general anaesthetic is being administered to a patient who is breathing spontaneously there is always a risk of upper airway obstruction. Such obstruction is dangerous and potentially fatal. It may be caused by the tongue falling into the hypopharynx or by the structures of the pharynx collapsing as a result of the loss of normal muscle tone.

One widely used device to prevent such obstruction from occurring and allow the continued smooth administration of oxygen with or without anaesthetic gases is the reusable laryngeal mask airway. One type of laryngeal mask airway is the reinforced laryngeal mask. An advantage of this particular device is that the patient may be allowed to regain consciousness with the device in situ. This helps ensure that the upper airway remains open and permits the continued administration of oxygen in the recovery room. One of the problems of the use of such a device, however, is that during emergence from anaesthesia, but before consciousness has been regained, the patient almost invariably bites on the reinforced flexible tube component of the laryngeal mask airway at the point where it passes between his or her incisors due to the (unconscious) physiological bite reaction. There are two serious consequences of this. First, the flow of oxygen to the patient is obstructed, frequently resulting in the saturation of haemoglobin with oxygen in the patient's blood falling to undesirable or even dangerous levels. Secondly, the reinforced laryngeal mask airway becomes damaged and is not thereafter available for further use as intended.

The problems associated with the patient's unconscious bite reaction on regaining consciousness have been recognised, and various bite protector devices have been devised. One prior art bite protector device is described in US patent 4425911 which describes a bite-block for tracheal tubes which comprises a substantially rectangular body having a central channel which is upwardly open and a pair of side members shaped to match the curve of the mouth and having surfaces shaped to engage the teeth other than the incisors. In use the bite block is inserted into the patient's mouth with the lateral wing portions engaged between the patient's molars such that the patient's incisors are held spaced apart.

Airway tubes, such as endotracheal tubes, suction catheters and the like are then introduced longitudinally through the channels in the bite protector and are protected from damage inflicted by the patients bite reaction due to the presence of the lateral wings which effectively prevent the molars from approaching one another and, therefore, hold the incisors spaced by a sufficient distance to prevent them from contacting the tubes. This bite protector works effectively for certain medical practices, but has a number of disadvantages in others. In particular, because it has to be fitted in position before the airways are introduced, it limits the available range of the medical practices to those in which the distal ends of the airway tubes are not equipped with anything larger than the cross-sectional dimension of the channel in the bite protector. In particular, therefore, such a bite protector cannot be used with a laryngeal mask airway as the laryngeal mask, which is permanently connected, as discussed above, to the distal end of the airway tube, is of sufficient dimensions to occupy the entirety of the patient's larynx lumen. This is considerably larger than the cross-sectional dimension of the airway passage.

Moreover, when utilising a laryngeal mask airway this may frequently be left in place whilst the patient regains consciousness in order to allow administration of oxygen. For access to the patient's mouth, and for the patient's comfort, it is necessary to be able to remove the bite protector from the patient's mouth whilst leaving the laryngeal mask in place. Such manipulation would be entirely impossible with the bite block described in US 4425911.

Another prior art endotracheal tube bite block is described in US 4896667. This has a simple design comprising a body with an elongate C-shape cross-section and comprises a core of a hard material surrounded by a material which is less hard than the core, the softer material being secured to a portion of the exterior of the hard core, and a portion of the hard core being exposed. This bite block has no face flange like the bite block of US 4425911 and it is described that such bite block may be secured to an endotracheal tube by taping the exposed portion of the hard core to the tube.

A longitudinal slot extends the length of the body, and radial apertures are provided for receiving locating straps to secure the bite block in position at a determined point along an endotracheal tube. Although the longitudinal slot would allow the introduction longitudinally of a narrow member it will not allow lateral introduction of a flexible airway such as to permit positioning of the bite block after the airway has been positioned within the patient's throat, nor to allow subsequent removal of the bite block whilst leaving the airway tube in position.

The present invention seeks to provide a bite protector which is suitable for use in particular with a reinforced laryngeal mask airway, but which may be used with other intubating airways for medical practices which do not require the laryngeal mask.

According to one aspect of the present invention, therefore, there is provided a bite protector for an airway, comprising an elongate body having a longitudinally extending passage therethrough with a discrutinuity extending along the wall at one side of the body the other wall being framed or provided with a hinge and there being a flange at one end provided to limit the distance the bite protector can be introduced into the mouth of a patient and a reinforced wall portion at least in the region adjacent the flange for resisting the bite reaction of a patient upon recovery from anaesthesia, the hinge being such as to allow the body to be opened laterally to an extent such that opposite edges of the discontinuity are spaced from one another by a distance at least as great as the maximum diameter of the passage through the body when closed.

In this respect the discontinuity is preferably an elongate slot and the closed position of the elongate body occurs when the opposite edges of the slot are in contact with one another. In this position the body is relaxed (that is unstressed) and in preferred embodiments of the invention the opposite edges of the slot have respective co-operating interengaging shapes to ensure a secure relative location. Such co-operating interengaging shape may include a V groove in the edge along one side of the slot with an appropriately shaped ridge along the other edge of the slot which ridge engages in the groove by form engagement when the slot is closed. Other shapes, including labyrinthine shapes, may be provided to ensure secure interengagement of the edges of the slot.

In one embodiment of the invention the end of the slot remote from the flange (hereinafter referred to as the distal end, whereas the end provided with the flange will be referred to as the proximal end) has an atraumatic curvature of the end portion of the wall whereby to avoid damage to a patient's palate upon introduction and/or removal.

Preferably the said face flange is formed into parts, one on each side of the hinge of the body, the two parts being shaped such as to limit the introduction of the body into the patient's mouth without restricting the opening movement of the body about the hinge thereof.

The reinforced wall portion in the region adjacent the face flange may be thicker than the remainder of the body, in which case it is preferred that the thicker portion is enlarged outwardly of the body, that is the internal surface of the thicker portion is coplanar with the internal surface of the remaining part of the body and the external surface of the thicker part meets the external surface of the remaining part of the body at a shoulder.

Other forms of reinforcement may, of course, be provided either as an alternative or in addition to the inspissation of the wall portion adjacent the face flange. In particular, an insert of a more resistant material may be introduced to, or co-moulded with, the body at or adjacent the face flange.

The shape of the body in cross-section may be anything suitable for the specific purpose. In particular, the body may have an approximately rectangular cross-section with a substantially flat lingual surface. An opposite, palatal surface may be convexly curved . Alternatively, opposite surfaces of the body may be flat, particularly in the region which in use will be contacted by the patient's incisors, or the body may be circular or substantially circular in cross-section throughout its length.

The present invention also comprehends an assembly comprising a laryngeal mask airway having an elongate flexible airway tube, an inflatable laryngeal mask at a distal end thereof, an inflation tube, and a bite protector as defined hereinabove, the internal diameter of the body of the bite protector being substantially the same as, and at least not less than, the outer diameter of the flexible airway tube.

Embodiments of the present invention will now be more particularly described, by way of example, with reference to the accompanying drawings, in which:
Figure 1 is a schematic view of a laryngeal mask airway provided with a bite protector as defined herein;
Figure 2 is a schematic perspective view of the bite protector shown separated from the laryngeal mask airway and in a closed, relaxed position; and
Figure 3 is a schematic perspective view of the bite protector of Figure 2, shown in an open position.

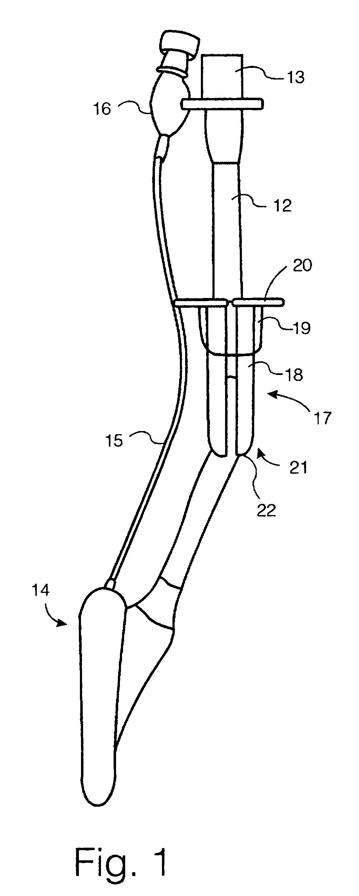

Referring now to the drawings, Figure 1 illustrates a laryngeal mask airway of known form comprising a flexible tube 12 having a connector 13 at a proximal end thereof, and a laryngeal mask generally indicated 14 at the distal end. The tube 12 is relatively soft and flexible in order to allow it to be introduced through a patient's mouth and into the pharynx where the mask 14 may be inflated via a separate inflation line 15 connected at its distal end to the mask 14 and having at its proximal end a bulb inflator 16 of known type. Injection of air through the bulb 16 causes the mask 14 to inflate and fill the pharynx thereby preventing air from escaping from the lungs around the outside of the tube 12, and correspondingly acting to prevent saliva and other liquids from draining into the lungs during medical operations. The bulb 16 includes valves (not shown) for controlling delivery of air to the line 15, and also for allowing deflation of the mask 14 when it is desired to remove it.

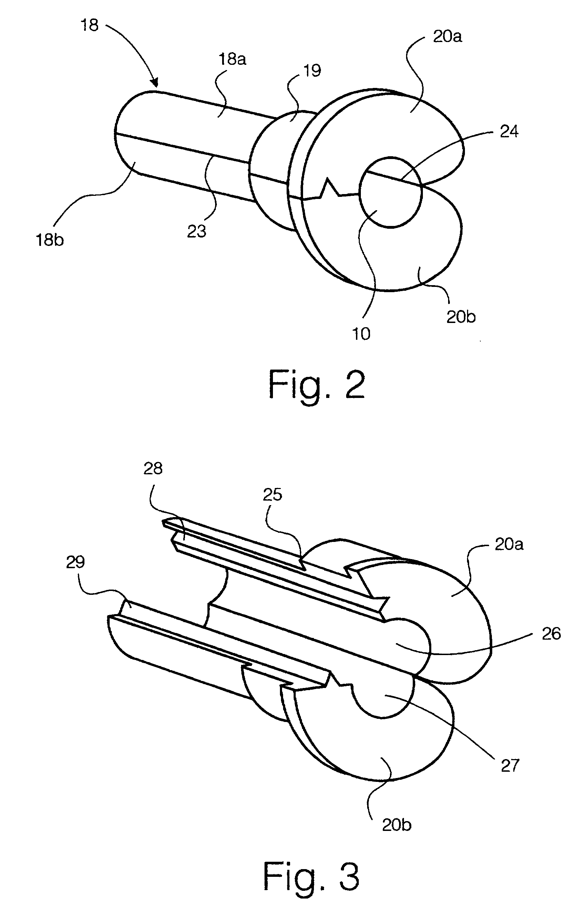

Around the flexible tube 12 is fitted a bite protector generally indicated 17 comprising an elongate generally tubular body 18 having a central passage 10 and an inspissated wall portion 19 adjacent a face flange 20 at a proximal end thereof. The tubular body 18 is straight but in alternative embodiment (not shown) may have a slight curve at the distal end. In the embodiment shown ???? 21 has rounded atraumatic shoulders 22 which ensure that the bite protector can be introduced into the mouth of a patient and, in particular, approach the throat without causing damage.
As can be seen in Figures 2 and 3, the body 18 is longitudinally split along a discontinuity or separation line 23 which may be considered as a slot extending the full length of the body, and diametrically opposite the separation line 23 the body has a hinge 24 extending parallel to the slot 23 and, with the slot 23, separating the body 18 into upper and lower halves 18a, 18b and, likewise, separating the face flange 20 into upper and lower halves 20a, 20b.

In this embodiment the body 18 has a circular cylindrical outer surface and a circular inner surface of corresponding form. The inspissated section meets the remaining part of the body at a shoulder 25 and the separation line or slot 23 has two opposite mating edges formed at the free edge of the upper body portion 18a and defining a V-section channel 28, whilst the opposite free edge of the lower body portion 18b forms a correspondingly shaped elongate ridge 29 which, when the body is in the closed relaxed position as shown in Figure 2 enters the groove 28 to form a secure interengagement holding the body 18 closed and resistant to any action tending to cause the edges 28, 29 of the slot 23 to become displaced from one another. In other embodiments (not shown) the body may have a different cross sectional shape, such as rectangular with flat opposite faces.

In use of the equipment the laryngeal mask 14 at the distal end of the flexible tube 12 is introduced into the patient's mouth and positioned in the region of the larynx and then inflated by use of the bulb 16. The bite protector 17 is opened as shown in Figure 3 and fitted over the flexible tube 12, following which it can be displaced longitudinally of the tube 12 into the patient's mouth with the inspissated section 19 in contact with the patient's incisors and the face flange 20 in contact with the patient's lips thereby defining a maximum insertion of the bite protector 17 into the patient's mouth.

The bite protector does not, in fact, have to be fitted until shortly before the patient is expected to arouse from the anaesthetic, and therefore does not have to be in place during initial administration of anaesthetic or during the operation where it may obstruct any manipulations of the flexible tube 12 or laryngeal mask 14. Once positioned, however, the bite protector offers a strong and robust protection against the jaw spasmic bite reaction which occurs unconsciously during the time a patient is recovering from anaesthesia. After recovery, however, the bite block can be removed readily by displacing it longitudinally of the flexible tube 12, opening it to the position shown in Figure 3 and removing it laterally from the tube 12 without disturbing it or applying any force which could cause disturbance to the location of the laryngeal mask 14.

The inspissated section 19 is, as can be seen from the drawings, of considerable thickness in relation to the thickness of the body 18, and if the entire bite protector is made from an appropriate plastics material, for example polypropylene or polyethylene, this thickness will provide sufficient strength to resist the unconscious bite reaction. Additional strength may be provided by fitting an external sleeve of stronger material over the inspissated section 19, or by co-moulding an insert into it. Such insert may, for example, be of metal or other more resistant plastics.

After use the bite protector can be discarded. Although it would be possible to make reusable bite protectors the necessity for sterilisation would involve a more complex structure than the simple moulded structure described, particularly since the hinge 24 which, in the embodiment described, is a simple polypropylene ligament hinge, would need to be of more complex structure. Disposable bite protectors are expected to be more hygienic and safer than reusable ones.

## Claims

1. A bite protector (17) for an airway comprising an elongate body (18) having a longitudinally extending passage (10) therethrough characterised in that it has a discontinuity (23) along the wall at one side of the body (18) and a hinge (24) along the other, a flange (20) at one end to limit the distance the bite protector (17) can be introduced into the mouth of a patient and a reinforced wall portion (19) at least in the region adjacent the flange (20) for resisting the bite reaction of a patient upon recovery from anaesthesia, the hinge (24) being such as to allow the body (18) to be opened laterally to an extent such that the opposite edges of the discontinuity (27) are spaced from one another by a distance at least as great as the maximum diameter of the passage (10) through the body (18) when closed.

2. A bite protector according to Claim 1, characterised in that in this the end (21) of the body (18) remote from the flange (20) has an atraumatic curvature (22) of the end portion of the wall whereby to avoid damage to a patient's palate upon introduction and/or removal.

3. A bite protector according to Claim 1 or Claim 2, characterised in that the said flange (20) is formed in two parts, one on each side of the hinge (24), the two parts being shaped such as to limit introduction into the patient's mouth without restricting the opening movement of the body (18) about the hinge (24) thereof.

4. A bite protector according to any preceding claim, characterised in that the said reinforced wall (19) is a thicker portion the internal surface of which is coplanar with the internal surface of the remaining part of the body.

5. A bite protector according to any preceding claim, characterised in that the reinforced wall portion (19) has an insert of stronger material inserted or co-moulded therein.

6. A bite protector according to any preceding claim, characterised in that the said body (18) has opposite substantially parallel wall portions in the region of the said reinforced wall portion (19) to be engaged by the incisors of a patient during recovery from anaesthesia.

7. A laryngeal mask airway having an elongate flexible airway tube (12), an inflatable laryngeal mask (14) at a distal end thereof, an inflation tube, and a bite protector according to any preceding claim, characterised in that the internal diameter of the body (18) of the bite protector is substantially the same as the outer diameter of the flexible airway tube (12).
